# FASCICULE DE BREVET EUROPEEN

(11) **EP 2 904 999 B1**
(45) Date de publication et mention de la délivrance du brevet: **15.02.2017**
(21) Numéro de dépôt: 15153133.2
(22) Date de dépôt: 29.01.2015
(51) Int. Cl.: A61F 13/08, A41B 9/06, A41D 13/00, D04B 1/24

(54) **Textile pour vêtements contre une lipodystrophie superficielle.**
Textilie für Kleidungsstücke gegen eine oberflächliche Lipodystrophie
Fabric for garments to prevent surface lipodystrophy

(30) Priorité: 29.01.2014 FR 1450695
(43) Date de publication de la demande: 12.08.2015
(73) Titulaire: BV Sport, 42000 Saint Etienne (FR)
(72) Inventeur: Corona, Salvator, 42350 LA TALAUDIERE (FR); Corona, Nicolas, 42000 SAINT ETIENNE (FR); Couzan, Serge, 42000 SAINT ETIENNE (FR); Pouget, Jean François, 42580 L'ETRAT (FR)
(74) Mandataire: Delorme, Nicolas

(56) Documents cités:
- WO-A1-2005/006894
- WO-A1-2012/109474
- WO-A2-2007/059046
- FR-A1- 2 865 905
- FR-A1- 2 992 150

## Description

La présente invention concerne un textile destiné à la confection de vêtements de compression pour lutter contre une lipodystrophie superficielle, notamment durant la pratique d'une activité physique de l'utilisateur. La présente invention concerne également un vêtement de compression comprenant un tel textile et adapté pour effectuer un massage de type palper/rouler sur l'utilisateur.

La lipodystrophie superficielle ou PEFS (Panniculopathie oedèmato-fibro-sclérotique) (ou encore cellulite dans le langage courant) correspond à une hypertrophie de la graisse localisée sous la peau, dans l'hypoderme, associée à une rétention d'eau et de toxines. Elle donne un aspect de 'peau d'orange' typique et disgracieux à la peau. Afin de lutter contre la cellulite, de nombreux vêtements de sport ont été proposés. Une grande partie de ces vêtements est basée sur la sudation qui reste toutefois inefficace pour traiter l'hypertrophie des adipocytes. D'autres vêtements sur le marché utilisent un textile dont la face en contact avec la peau comprend des microbilles pour un effet massant. Des vêtements permettant la diffusion de produits thérapeutiques anticellulite par micro-encapsulation sont également disponibles. Néanmoins, la réduction de la cellulite par ce biais n'est pas encore clairement démontrée. Des cuissards formés par un textile composé d'une enveloppe tricotée élastique sur la face intérieure et d'une face extérieure adaptée à la contention avec un gradient dégressif de pression sont également connus pour lutter contre la cellulite. La face extérieure de ce cuissard comporte également des éléments en relief tel que des microbilles afin d'augmenter la compression effectuée par le vêtement. Cependant, même si ce type de cuissard est efficace pour le drainage lymphatique, son action massante et d'élimination de la cellulite reste limitée.

Un des buts de la présente invention vise alors à pallier ces inconvénients en proposant une solution pour lutter efficacement contre la cellulite. Pour ce faire, l'invention concerne un textile destiné à la confection de vêtements de compression pour lutter contre une lipodystrophie superficielle, le textile comprenant une pluralité d'alternances de régions de pression et de régions de dépression, les régions de pression étant aptes à comprimer des régions de tissus biologiques d'un utilisateur et les régions de dépression étant aptes à relâcher des régions de tissus biologiques de l'utilisateur, les régions de pression présentant une résistance deux fois plus importante à l'étirement que les régions de dépression et la pluralité d'alternances étant conformées de sorte à réaliser un massage lors de mouvements musculaires de l'utilisateur, en particulier un massage de type palper/rouler.

Selon une autre possibilité, l'invention concerne un textile destiné à la confection de vêtements de compression pour lutter contre une lipodystrophie superficielle, le textile comprenant une pluralité d'alternances de régions de pression et de régions de dépression, les régions de pression étant aptes à comprimer des régions de tissus biologiques d'un utilisateur et les régions de dépression étant aptes à relâcher des régions de tissus biologiques de l'utilisateur, les régions de pression présentant une résistance à l'étirement plus importante que les régions de dépression et la pluralité d'alternances étant conformées de sorte à réaliser un massage de type palper-rouler lors de mouvements musculaires de l'utilisateur.

Selon un mode de réalisation, les régions de pression présentent une résistance à l'étirement compris entre 1,5 et deux fois celle des régions de dépression.

Par l'expression 'tissus biologiques', on entend dans ce document la peau, les tissus graisseux, les muscles, les tendons, les nerfs, les vaisseaux tels que les artères ou veines lymphatiques. Ainsi ce type de textile, utilisé par exemple dans un vêtement tel qu'un cuissard, permet l'application de pressions différenciées et graduelles sur des régions localisées de la peau qui se répercutent sur les amas graisseux de l'hypoderme.

Associées à une contraction musculaire, ces différences de pression permettent la création d'ondulations de la peau et de la graisse au niveau des régions de dépression. Lorsque le muscle est décontracté, les régions relâchées des tissus se tendent et les ondulations sont nivelées. La mise en mouvement du bassin, de la cuisse et du genou entraine un jeu articulaire provoquant des allongements et raccourcissements répétés des tissus graisseux. Le mouvement d'ondulation de la peau qui en résulte peut être comparé au pincement dans la technique de massage de type palper/rouler. Réciproquement, le nivellement de la peau peut être comparé au roulement dans la technique de massage de type palper/rouler. Ainsi, à chaque contraction et décontraction musculaire et du fait du jeu articulaire, une action de pincement et de roulage est effectuée sur l'amas graisseux par le textile, ce qui permet sa fragmentation et son élimination.

Ce textile permet ainsi une fragmentation et une élimination de cellulite comparable à celle obtenue par un massage de type palper/rouler tout en favorisant un excellent drainage lymphatique et une très bonne microcirculation.

Par ailleurs, ce textile destiné à des vêtements de compression permet d'accélérer le flux sanguin ce qui participe à l'évacuation naturelles des graisses dans les tissus.

Selon un mode de réalisation, le textile comprend au moins trois alternances de régions de pression et de régions de dépression.

Les régions de pression et régions de dépression du textile présentent une résistance différente à l'étirement qui est notamment obtenue par un maillage de fibres, dits « maillages à allongement faible » ou « maillages à allongement long ». Les mailles à allongement faible étant obtenues par une augmentation localisée du facteur de couverture des mailles. Ceci contribue à réduire l'élasticité segmentaire des régions de pression du textile.

Selon une alternative, le maillage à allongement faible est obtenu par tricotage avec ajout fractionné à la trame de fils présentant une rigidité supérieure aux fils formant la trame d'un maillage à allongement long. Ceci contribue à réduire l'élasticité circonférentielle des régions de pression du textile.

Selon une autre alternative, le maillage à allongement faible est obtenu par ajout de fils présentant une rigidité supérieure aux fils formant la trame d'un maillage à allongement long. Cette méthode contribue également à réduire l'élasticité circonférentielle des régions de pression du textile.

Selon encore une autre alternative, le maillage à allongement faible est obtenu par couture ou collage d'éléments externes, tel que des éléments de silicones, destinés à bloquer les mailles à allongement long.

Avantageusement, chaque des régions de pression du textile présente une face comportant des éléments d'agrippement configurés pour agripper la région de pression respective du textile sur la région respective de tissus biologiques de l'utilisateur au cours de mouvements musculaires.

Le glissement des régions de pression du textile sur les tissus biologiques est ainsi évité. Il en résulte que l'effet de pincement des tissus obtenu est optimisé. Le textile permet ainsi d'effectuer un massage palper/rouler mécanique similaire au massage palper/rouler manuel, tel que pratiqué par les kinésithérapeutes.

De préférence, les éléments d'agrippement sont réalisés sous la forme d'au moins une bande en silicone.

Avantageusement, le textile de l'invention comprend une pluralité de bandes de silicone, chacune des bandes de silicone formant la totalité d'une région de pression.

Ainsi, il suffit d'une bande de silicone incorporée au textile pour le rigidifier localement et obtenir une région de pression, de la forme d'une bande, qui peut être utilisée pour former une région de pression annulaire dans un vêtement.

Selon une possibilité, les éléments d'agrippement sont formés par des picots aptes à pénétrer superficiellement dans les tissus biologiques de l'utilisateur. Ces picots peuvent être obtenus par modification de la trame du textile, par ajout de silicone ou par ajout de polyuréthane. Ces picots permettent ainsi de renforcer l'action de pincement des tissus adipeux sous-jacents aux régions de pression du textile (régions comprimées). La contraction musculaire entraine un mouvement de rapprochement par translation des régions comprimées pincées par les régions de pression. Il en résulte la formation d'une ondulation des régions de tissus adipeux sous-jacentes aux régions de dépression du textile, situées entre deux régions pincées. Puis la décontraction musculaire entraine un mouvement d'écartement des régions comprimées pincées de sorte à tendre l'ondulation des régions relâchées. Ces mouvements répétés et optimisés par la présence de picots amplifient la fragmentation de l'amas graisseux et son élimination.

Selon une variante de réalisation, les éléments d'agrippement sont réalisés par un réseau de protubérances comprenant une pluralité de lignes de protubérances s'étendant selon une première direction, les protubérances de deux lignes adjacentes étant disposées en quinconce.

Ces protubérances organisées en réseau permettent aux régions de pressions du textile d'agripper de façon durable la peau d'un utilisateur. Ainsi, cette configuration favorise le maintien des régions de pressions en place et limite le glissement des régions de pression sur la peau. Il s'ensuit que le pincement et le roulement des tissus biologiques lors des mouvements musculaires de l'utilisateur est amélioré.

Les protubérances sont avantageusement formées par modification de la trame du textile ou par ajout de silicone.

Avantageusement, pour chaque ligne, les protubérances sont espacées d'environ 2 mm.

De préférence, chaque protubérance présente une dimension d selon la première direction X d'environ 4 mm et une dimension d' selon une seconde direction sensiblement perpendiculaire à la première dimension X d'environ 2,5 mm.

Selon une possibilité, deux lignes adjacentes et s'étendant selon la première direction présentent une distance d'environ 3 mm.

Selon une disposition, l'épaisseur du textile à chacune des protubérances est d'environ 2,5 mm, l'épaisseur du textile dépourvu de protubérances est comprise entre 1,5 et 2 mm environ.

Selon une possibilité, chaque région de dépression et chaque région de pression du textile présente une périphérie comprenant principalement des portions curvilignes.

Selon une disposition, chaque région de dépression et chaque région de pression présente une périphérie comprenant principalement des portions rectilignes.

Selon une autre disposition, la plus grande dimension des régions de dépression est comprise entre 1 et 3 cm.

Selon une autre possibilité la plus grande dimension des régions de pression est comprise entre 0,5 et 4 cm.

Le choix adapté des dimensions des régions de compression et de dépression permettent d'optimiser l'action sur les tissus biologiques notamment par l'obtention d'un nombre d'alternance important et contrôlée des régions comprimées ou relâchées.

Avantageusement, la plus petite dimension des régions de pression est comprise entre 0,5 et 2,5 cm. Ces régions de pression sont notamment utilisées pour former des bandes annulaires dans un vêtement, par exemple un cuissard, qui viendront pincer la peau de l'utilisateur entre deux bande annulaires pour un massage palper-rouler lors des mouvements musculaires de l'utilisateur.

De préférence, la plus petite dimension des régions de dépression est comprise entre 1 et 2,5 cm. Il est ainsi possible d'utiliser les régions de dépression pour former une bande annulaire dans le vêtement. La bande annulaire facilite ainsi la formation d'un pli dans la peau relâchée de l'utilisateur par pincement entre deux régions de pression annulaires.

Selon une disposition, chacune des régions de pression et chacune des régions de dépressions présente la forme d'une bande. Il va s'en dire qu'il est entendu dans la présente demande que la bande est rectiligne ou sinusoïdale. Ainsi, lors de l'utilisation d'un tel textile dans un vêtement, un pli cutané peut être obtenu lors de mouvements musculaires de l'utilisateur.

Avantageusement, le textile comprend des fibres à base de polymère, tel que du polyamide, de l'élasthanne et du polyester.

De préférence, au moins une pluralité d'alternance de régions de dépression et de régions de pression est tricotée sur un métier à tricoter circulaire à grand cylindre en mode coupé cousu.

Selon une alternative, une alternance de régions de dépression et de régions de pression est tricotée sur un métier à tricoter circulaire à petit cylindre, notamment pour fabriquer des vêtements de petites dimensions, telles que des manchons.

Selon une possibilité, au moins une alternance de régions de dépression et de régions de pression est tricotée d'un seul tenant. Ceci permet d'éviter toute couture entre les différentes régions de sorte à améliorer le confort de l'utilisateur. Ceci est notamment utile dans le cas de fabrication de vêtement à application médicale.

Selon un second aspect, la présente invention concerne un vêtement de compression adapté pour effectuer un massage sur un utilisateur, notamment un massage de type palper/rouler le vêtement comprenant au moins une portion formée d'un textile tel que précédemment décrit. Ainsi, ce type de vêtement de compression permet un processus de lipolyse ou de destruction de la graisse inutile ainsi que l'accélération du flux sanguin aidant I évacuation naturelles des graisses tout en activant, en profondeur et en superficie, la circulation artérielle, veineuse et lymphatique. Ceci permet par ailleurs de maintenir les fibres musculaires, de diminuer le risque de blessures et d'améliorer la récupération du l'utilisateur.

Selon un mode de réalisation, le vêtement de compression comprend au moins trois alternances de régions de pression et de régions de dépression dans la direction longitudinale et dans la direction verticale du vêtement de compression.

De préférence, les régions de pression du textile sont aptes à comprimer des régions des tissus biologiques de l'utilisateur avec une pression inférieure ou égale à 15 mm Hg et de préférence comprise entre environ 8 et 12 mm Hg. Cette pression est calculé selon la loi de Laplace exprimant une relation inversement proportionnelle entre la pression appliquée et le rayon de courbure des tissus biologiques. Associé à l'activité physique, ce vêtement de compression augmente ainsi de manière considérable le pouvoir de destruction des adipocytes déformables à l'origine de la peau d'orange. Ceci est réalisé de manière naturelle, sans aucun ajout de produit ou traitement et uniquement en optimisant la physiologie de la personne en mouvement.

Avantageusement, les régions de dépression sont aptes à relâcher des régions des tissus biologiques de l'utilisateur avec une pression comprise entre environ 3 et 7 mm Hg. Ceci assure un relâchement suffisant des tissus pour permettre leur ondulation et l'obtention d'une action de type massage de palper/rouler.

De préférence, la pression appliquée par les régions de pression est environ le double de la pression appliquée par les régions de dépression pour une efficacité optimale.

Avantageusement, les pluralités d'alternance des régions de pression et des régions de dépression sont configurées pour comprimer les tissus biologiques de l'utilisateur avec une pression comprise entre 3 et 15 mm Hg et de préférence entre 7 et 9 mm Hg.

Ainsi, les mouvements musculaires de l'utilisateur portant un vêtement conçu selon la présente invention permettent le pincement des tissus pour un massage palper-rouler mécanique de sorte à fragmenter l'amas graisseux à l'origine de la cellulite. En deuxième effet, la compression des tissus améliore le flux sanguin et augmente l'efficacité du drainage des fragments de capitons obtenus et facilite son élimination.

Selon une disposition, le textile de la au moins une portion est obtenu par tricotage circulaire de sorte que la au moins une portion est exempte de couture. Le vêtement de compression est ainsi confortable à porter.

Selon une alternative, la au moins une pluralité d'alternance de régions de dépression et de régions de pression est tricotée sur un métier à tricoter circulaire à grand cylindre en mode coupé cousu. Les portions de textile sont alors cousues entre elles par une couture, notamment dans le cas d'un manchon ou à l'entrejambe dans le cas d'un cuissard.

Selon une autre possibilité, la au moins une pluralité d'alternance de régions de dépression et de régions de pression est tricotée sur un métier à tricoter circulaire petit cylindre. Ceci est avantageusement le cas lors de la fabrication de textile destiné à des vêtements de petites tailles de grand bien être s'il n'y a pas de couture, telle que des manchons par exemple.

Selon encore une autre alternative, les régions de pression sont tricotées ou tissées indépendamment des régions de dépression avant assemblage les unes avec les autres. Ce mode de réalisation est avantageusement mis en oeuvre lorsque les régions de pression et les régions de dépression s'étendent principalement dans une direction en formant une bande. Ceci est notamment utile pour former des bandes annulaires dans un vêtement.

Avantageusement, le vêtement de compression est un cuissard, un manchon de bras, un manchon de cuisse ou un collant. Il est ainsi possible de traiter efficacement l'aspect peau d'orange localisé, en améliorant le processus de phagocytose du tissu graisseux piégé, inutile et inesthétique, tout en améliorant la tonicité musculaire et tissulaire pendant l'activité physique. En cas de port régulier, la production cellulaire de collagène et d'élastine est relancée. Ainsi, les volumes graisseux compressés sont remodelés, la silhouette s'affine, se lisse et reprend des contours harmonieux.

Par ailleurs, le cuissard permet de solliciter les muscles lents comme les fessiers et d'appliquer des pressions sur la sangle abdominale, ce qui permet une correction posturale.

Selon une disposition particulière, les régions de pression présentent chacune une forme globalement annulaire.

Selon une disposition, les régions de pression annulaires peuvent s'étendre selon une direction en biais par rapport à la direction longitudinale du vêtement de compression.

Pour un effet maximal, les régions de pression annulaires s'étendent selon une direction sensiblement perpendiculaire à la direction longitudinale du vêtement de compression. Ainsi, les régions de pression annulaires sont orientées de façon sensiblement perpendiculaire au fibres musculaires visées pour le massage palper-rouler.

Selon une autre disposition particulière, les régions de dépression présentent chacune une forme globalement annulaire.

De préférence, les régions de dépression annulaires s'étendent selon une direction perpendiculaire à la direction longitudinale du vêtement de compression.

D'autres aspects, buts et avantages de la présente invention apparaîtront mieux à la lecture de la description suivante de différents modes de réalisation de celle-ci, donnée à titre d'exemples non limitatifs et faite en référence aux dessins annexés. Les figures ne respectent pas nécessairement l'échelle de tous les éléments représentés de sorte à améliorer leur lisibilité. Dans la suite de la description, par souci de simplification, des éléments identiques, similaires ou équivalents des différentes formes de réalisation portent les mêmes références numériques.
La figure 1 illustre un textile selon un premier mode de réalisation de l'invention.
La figure 2 illustre un textile selon un deuxième mode de réalisation de l'invention.
La figure 3 illustre un textile selon un troisième mode de réalisation de l'invention.
La figure 4 illustre un vêtement de compression selon un mode de réalisation de l'invention.
Les figures 5 et 6 illustrent l'action d'un vêtement de compression selon l'invention sur des tissus biologiques.
La figure 7 illustre un mode de réalisation des éléments d'agrippement selon l'invention.
La figure 8 illustre un deuxième mode de réalisation des éléments d'agrippement selon l'invention.

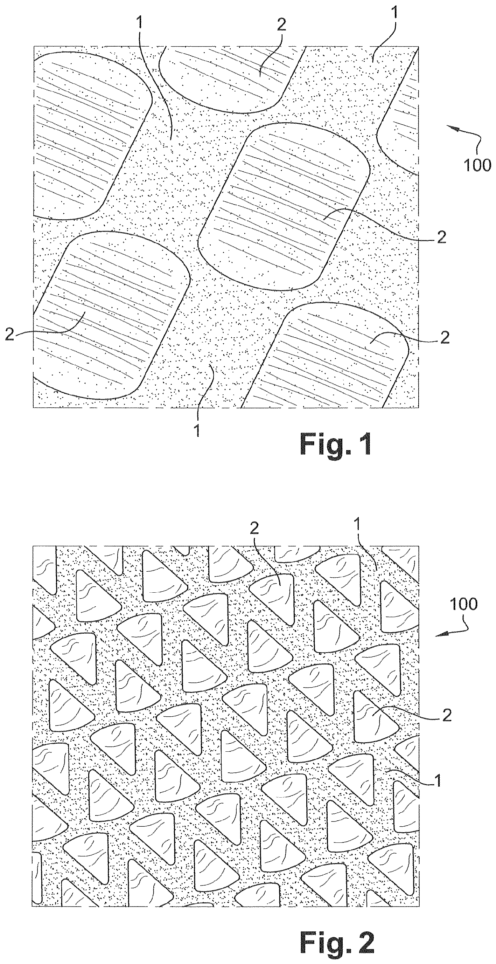

Comme illustré à la figure 1, le textile 100 présente des régions de pression 1 et des régions de dépression 2 régulièrement alternées. Les régions de pression 1, aptes à comprimer des tissus biologiques, présentent une résistance à l'étirement deux fois plus importante que celle des régions de dépression 2. Ces deux types de régions 1,2 sont tricotés d'un seul tenant de sorte à ne pas nécessiter la présence de coutures ou d'ourlets entre chacune des régions 1,2. Les fibres constituant le textile 100 sont des fibres à base de polyamide, élasthanne et polyester. Selon une possibilité, la composition des fibres est de 30% de polyamide 10% d'élasthanne et de 60 % de polyester.

Dans ce mode de réalisation, les régions de pression et de dépression 1,2 présentent une périphérie comprenant principalement des portions globalement curvilignes. La plus petite dimension des régions de pression 1 est comprise entre 1 et 2.5 cm. Ces dimensions sont adaptées au pincement efficace des tissus biologiques au cours de mouvements d'un utilisateur. La plus petite dimension des régions de dépression 2 est comprise entre 0,5 et 2.5 cm ce qui permet aux régions relâchées des tissus d'onduler et de s'étirer, favorisant la dégradation de l'amas graisseux 5 sous-jacent. De plus, ces dimensions limitent l'espacement entre les régions de pression 1 de sorte à obtenir un effet de pincement et de massage optimal.

La figure 2 illustre un deuxième mode de réalisation du textile 100 selon l'invention dans lequel les périphéries des régions de pression 1 et des régions de dépression 2 présentent majoritairement une forme rectiligne. La plus grande dimension des régions de pression 1 est comprise entre 0.5 et 4 cm tandis que la plus grande dimension des régions de dépression 2 est comprise entre 1 et 3 cm.

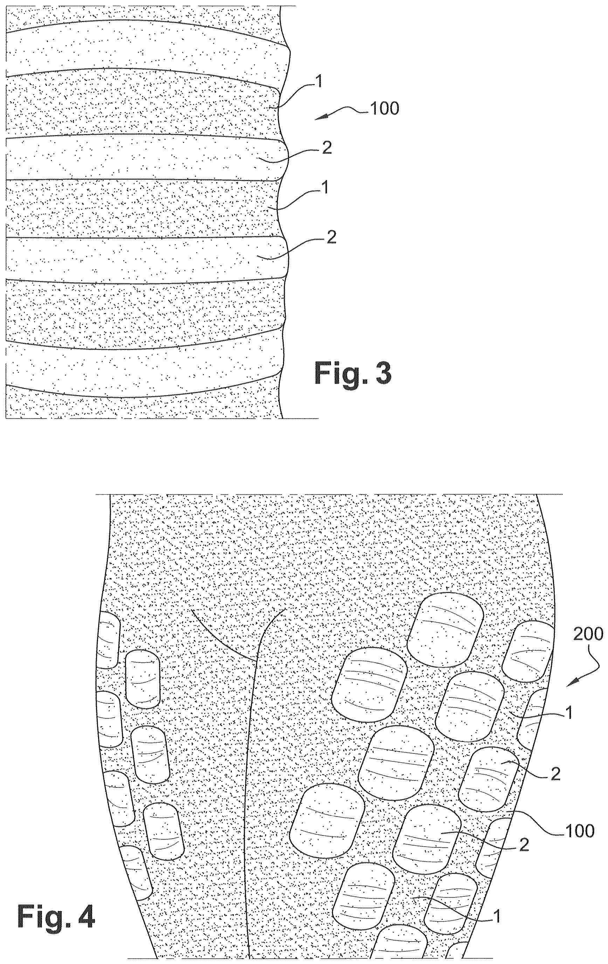

La figure 3 illustre un troisième mode de réalisation du textile 100 différent du précédent en ce que les régions s'étendent de façon plus importante. La plus petite dimension des régions de dépression 2 et de pression est d'environ 2.5 cm tandis que la plus grande dimension est celle du textile 100. Ce type de textile est apte à former des vêtements aux régions de pression 1 et des régions de dépression 2 annulaires, particulièrement bien adaptées pour opérer un pincement /roulement des tissus, lors de mouvement musculaires (marche, sport...) et l'obtention du palper-rouler du kinésithérapeute.

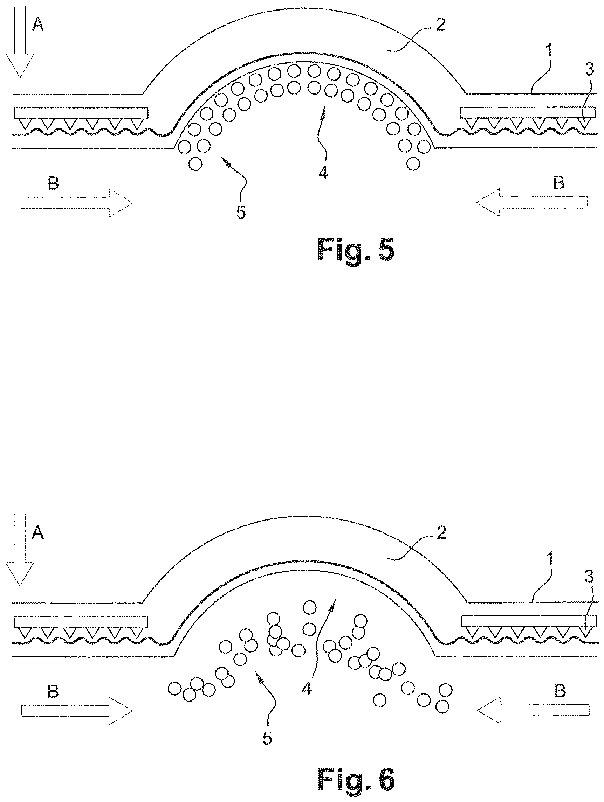

Selon une disposition, les régions de pression 1 présentent une face comportant des picots 3 aptes à pénétrer en surface des tissus biologiques (figures 5 et 6). Ces picots 3 forment typiquement des éléments d'agrippement des régions de pression 1 sur les tissus biologiques d'un utilisateur. Ces éléments d'agrippement permettent d'éviter le glissement du textile 100 sur l'utilisateur, rendant encore plus efficace le pincement des tissus biologiques.

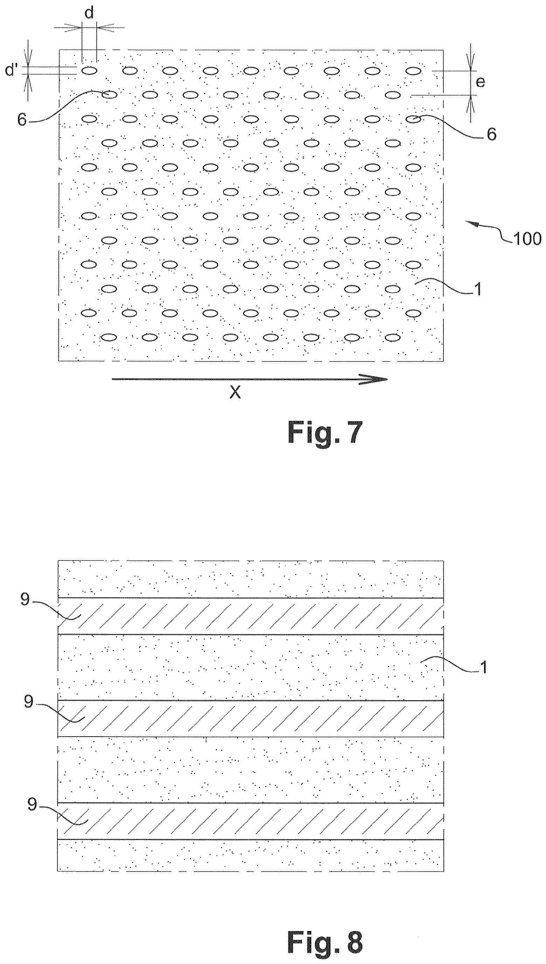

Comme illustré aux figures 5, 6, 7 et 8, un mode de réalisation avantageux prévoit que chaque région de pression du textile comprend des éléments d'agrippement. Comme illustré aux figures 5 et 6, ces éléments sont formés à partir de picots 3. La figure 7 illustre un autre mode de réalisation dans lequel les éléments d'agrippement sont réalisés par un réseau de protubérances 6 comprenant une pluralité de lignes de protubérances 6 s'étendant selon une première direction X. Il est à noter que les protubérances 6 de deux lignes adjacentes sont disposées en quinconce pour améliorer l'effet d'agrippement du textile. Les protubérances 6 de chaque ligne sont espacées d'environ 2 mm. Chaque protubérance 6 présente une dimension d selon la première direction X d'environ 4 mm et une dimension d' selon une seconde direction sensiblement perpendiculaire à la première direction X d'environ 2,5 mm.

Selon une autre variante illustrée à la figure 8, les éléments d'agrippement sont réalisés par des bandes 9 en silicone qui ont la propriété d'adhérer à la peau.

Selon un deuxième aspect illustré à la figure 4, l'invention concerne également un vêtement 200 de compression, tel qu'un cuissard, constitué d'au moins une portion formée du textile 100 tel que précédemment décrit et obtenu par un tricotage circulaire de sorte à éviter la présence de couture entre les régions de pression et de dépression 1,2. Cette portion de vêtement 200 de compression est ainsi dotée de régions de pression 1 et de régions de dépression 2 de sorte que le vêtement 200 de compression est adapté pour effectuer un massage au cours des mouvements de l'utilisateur qui le porte. Les régions de pression 1 présentent une forme globalement annulaire s'étendant selon une direction sensiblement perpendiculaire à la direction longitudinale du vêtement 200 de compression. Selon des variantes de réalisation non illustrées, le vêtement 200 de compression peut être un manchon de bras, un manchon de cuisse ou un collant et les régions de pression 1 et de dépression 2 peuvent prendre tout type de formes.

Le vêtement 200 de compression est conçu et dimensionné de sorte qu'une fois porté, il applique une pression d'environ sur les tissus biologiques correspondant, quels que soient la taille de l'utilisateur, en vue de pouvoir créer un effet de pincement comme illustré par l'ondulation 4 sur les figures 5 et 6.

La figure 5 illustre l'effet du vêtement 200 de compression sur les tissus biologiques d'un utilisateur. Les régions de pression 1 appliquent une pression (flèches A) plus importante que celle appliquée par les régions de dépression 2 du fait de leur différence de résistance à l'étirement. Lorsque les muscles de l'utilisateur commence à se contracter (flèches B) les régions de tissus biologiques comprimées sous les régions de pression 1 se rapprochent. En conséquence, les régions de tissus relâchées sous les régions de dépression ondulent entrainant également l'ondulation de l'amas graisseux 5. Ceci conduit ainsi au pincement des tissus relâchés 3 avec l'obtention du même effet que celui obtenu lors d'un massage palper/rouler. Lorsque la contraction musculaire est plus importante (figure 6), les régions sous compression se rapprochent encore provoquant un pincement intense permettant de fragmenter l'amas graisseux 5.

Une fois que le muscle se détend, le mouvement inverse se produit. La peau ondulée se retend et s'aplanit, entrainant l'amas graisseux 5 fragmenté qui est alors facilement éliminé par l'organisme.

Ce mouvement de pincement/ondulation est répété à chaque contraction musculaire de sorte que la fragmentation de l'amas graisseux 5 est répétitive et que son élimination devient visible, notamment par une disparition sensible de l'aspect 'peau d'orange'.

Ainsi, la présente invention apporte une amélioration déterminante à l'état de la technique antérieure en proposant un textile 100 et un vêtement 200 de compression comprenant au moins une portion de ce textile 100, adapté pour une action massante par pincement et roulement des tissus biologiques avec une efficacité similaire à celle d'un massage de type palper-rouler. La compression apportée par le vêtement 200 facilite ensuite le drainage et l'élimination des fragments de capitons obtenus lors du massage palper-rouler.

Il va de soi que l'invention n'est pas limitée aux modes de réalisation décrits ci-dessus à titre d'exemples mais qu'elle comprend tous les équivalents techniques et les variantes des moyens décrits ainsi que leurs combinaisons.

## Revendications

1. Textile (100) destiné à la confection de vêtements (200) de compression pour lutter contre une lipodystrophie superficielle, **caractérisé en ce que** le textile (100) comprend une pluralité d'alternances de régions de pression (1) et de régions de dépression (2), les régions de pression (1) étant aptes à comprimer des régions de tissus biologiques d'un utilisateur et les régions de dépression (2) étant aptes à relâcher des régions de tissus biologiques de l'utilisateur, les régions de pression (1) présentant une résistance à l'étirement entre 1.5 et 2 fois celle des régions de dépression (2) de sorte à réaliser un massage de type palper-rouler lors de mouvements musculaires de l'utilisateur.

2. Textile (100) selon l'une des revendications 1 à 2, dans lequel chaque région de pression (1) du textile présente une face comportant des éléments d'agrippement configurés pour agripper la région de pression (1) respective du textile (100) sur la région respective de tissus biologiques de l'utilisateur au cours de mouvements musculaires.

3. Textile (100) selon la revendication 3, dans lequel les éléments d'agrippement sont réalisés sous la forme d'au moins une bande (9) en silicone.

4. Textile (100) selon la revendication 3, dans lequel les éléments d'agrippement sont formés par des picots (3) aptes à pénétrer superficiellement dans les tissus biologiques de l'utilisateur.

5. Textile (100) selon la revendication 3, dans lequel les éléments d'agrippement sont réalisés par un réseau de protubérances (6), le réseau de protubérances (6) comprenant une pluralité de lignes de protubérances (6) s'étendant selon une première direction (X), les protubérances (6) de deux lignes adjacentes étant disposées en quinconce.

6. Textile (100) selon la revendication 6, dans lequel chaque protubérance (6) présente une dimension (d) selon la première direction (X) d'environ 4 mm et une dimension (d') selon une seconde direction sensiblement perpendiculaire à la première direction (X) d'environ 2,5 mm.

7. Textile (100) selon l'une des revendications 1 à 7, dans lequel la plus petite dimension des régions de dépression (2) est comprise entre 1 et 2,5 cm.

8. Textile (100) selon l'une des revendications 1 à 8, dans lequel la plus petite dimension des régions de pression (1) est comprise entre 0,5 et 2,5 cm.

9. Textile (100) selon l'une des revendications 1 à 9, dans lequel chacune des régions de pression et chacune des régions de dépression présente la forme d'une bande.

10. Textile (100) selon l'une des revendications 1 à 10, dans lequel au moins une pluralité d'alternance de régions de dépression (2) et de régions de pression (1) est tricotée sur un métier à tricoter circulaire à grand cylindre en mode coupé cousu.

11. Vêtement (200) de compression adapté pour effectuer un massage de type palper-rouler sur un utilisateur, **caractérisé en ce que** le vêtement (200) comprend au moins une portion formée d'un textile (100) selon l'une des revendications 1 à 11.

12. Vêtement (200) selon la revendication 12, dans lequel les pluralités d'alternance des régions de pression (1) et des régions de dépression (2) sont configurées pour comprimer les tissus biologiques de l'utilisateur avec une pression comprise entre 3 et 15 mm Hg et de préférence entre 7 et 9 mm Hg.

13. Vêtement (200) selon l'une des revendications 12 à 13, dans lequel le vêtement (200) est un cuissard, un manchon de bras, un manchon de cuisse, ou un collant.

14. Vêtement (200) selon l'une des revendications 12 à 14, dans lequel les régions de pression (1) présentent une forme globalement annulaire.

## Patentansprüche

1. Textil (100), bestimmt zur Herstellung von Kleidungsstücken (200) zur Kompression, um gegen oberflächliche Lipodystrophie anzukämpfen, **dadurch gekennzeichnet, dass** das Textil (100) eine Vielzahl von Wechseln zwischen Druckbereichen (1) und Unterdruckbereichen (2) aufweist, wobei Druckbereiche (1) geeignet sind, um biologische Gewebebereiche eines Benutzers zu komprimieren und die Unterdruckbereiche (2) geeignet sind, um biologische Gewebebereiche eines Benutzers zu entspannen, wobei die Druckbereiche (1) einen Widerstand gegen der Rückzug aufweisen, der zwischen 1,5 und 2 Mal derjenige der Unterdruckbereiche (2) ist, um eine Massage vom Typ Kneten-Rollen bei den Muskelbewegungen des Benutzers durchzuführen.

2. Textil (100) nach einem der Ansprüche 1 bis 2, wobei jeder Druckbereich (1) des Textils eine Seite aufweist, die Greifelemente umfasst, konfiguriert, um den entsprechenden Druckbereich (1) des Textils (100) auf dem entsprechenden biologischen Gewebebereich des Benutzers im Laufe von Muskelbewegungen zu greifen.

3. Textil (100) nach Anspruch 3, wobei die Greifelemente in Form von mindestens einem Silikonband (9) durchgeführt sind.

4. Textil (100) nach Anspruch 3, wobei die Greifelemente aus Picots (3) gebildet sind, die geeignet sind, um oberflächlich in die biologischen Gewebe des Benutzers einzudringen.

5. Textil (100) nach Anspruch 3, wobei die Greifelemente aus einem Netz von Vorsprüngen (6) durchgeführt sind, wobei das Netz von Vorsprüngen (6) eine Vielzahl von Linien mit Vorsprüngen (6) umfasst, die sich gemäß einer ersten Richtung (X) erstrecken, wobei die Vorsprünge (6) von zwei benachbarten Linien versetzt angeordnet sind.

6. Textil (100) nach Anspruch 6, wobei jeder Vorsprung (6) eine Dimension (d) in der ersten Richtung (X) von ungefähr 4 mm aufweist, sowie eine Dimension (d') in einer zweiten Richtung im Wesentlichen senkrecht zur ersten Richtung (X) von ungefähr 2,5 mm aufweist.

7. Textil (100) nach einem der Ansprüche 1 bis 7, wobei die kleinste Dimension der Unterdruckbereiche (2) im Bereich zwischen 1 und 2,5 cm liegt.

8. Textil (100) nach einem der Ansprüche 1 bis 8, wobei die kleinste Dimension der Druckbereiche (1) im Bereich zwischen 0,5 und 2,5 cm liegt.

9. Textil (100) nach einem der Ansprüche 1 bis 9, wobei jeder der Druckbereiche und jeder der Unterdruckbereiche die Form eines Bands aufweist.

10. Textil (100) nach einem der Ansprüche 1 bis 10, wobei mindestens eine Vielzahl des Wechsels zwischen Unterdruckbereichen (2) und Druckbereichen (1) auf einer runden Wirkmaschine mit großem Zylinder im Modus Geschnitten-Genäht gewirkt ist.

11. Kleidungsstück (200) zur Kompression, das ausgelegt ist, um eine Massage vom Typ Kneten-Rollen auf einem Benutzer auszuführen, **dadurch gekennzeichnet, dass** das Kleidungsstück (200) mindestens einen Abschnitt umfasst, der aus einem Textil (100) nach einem der Ansprüche 1 bis 11 gebildet ist.

12. Kleidungsstück (200) nach Anspruch 12, wobei die Vielzahlen von Wechsel zwischen Druckbereichen (1) und Unterdruckbereichen (2) konfiguriert sind, um die biologischen Gewebe des Benutzers mit einem Druck zu komprimieren, der im Bereich zwischen 3 und 15 mm Hg und vorzugsweise zwischen 7 und 9 mm Hg liegt.

13. Kleidungsstück (200) nach einem der Ansprüche 12 bis 13, wobei das Kleidungsstück (200) ein Schenkelgurt, eine Armmuffe, eine Schenkelmuffe oder eine Strumpfhose ist.

14. Kleidungsstück (200) nach einem der Ansprüche 12 bis 14, wobei die Druckbereiche (1) im Wesentlichen ringförmig sind.

## Claims

1. A fabric (100) intended to the manufacture of compression garments (200) in order to control a surface lipodystrophy, **characterized in that** the fabric (100) comprises a plurality of alternations of pressure regions (1) and depression regions (2), the pressure regions (1) being adapted to compress regions of biological tissues of a user and the depression regions (2) being adapted to release regions of biological tissues of the user, the pressure regions (1) having a resistance to stretching between 1.5 and 2 times that of the depression regions (2) so as to carry out a massage of the type palpate-roll during muscular movements of the user.

2. The fabric (100) according to any of claims 1 to 2, wherein each pressure region (1) of the fabric has a face including gripping members configured to grip the respective pressure region (1) of the fabric (100) on the respective region of biological tissues of the user during muscular movements.

3. The fabric (100) according to claim 3, wherein the gripping members are made in the form of at least one silicone strip (9).

4. The fabric (100) according to claim 3, wherein the gripping members are formed by pins (3) able to superficially penetrate the biological tissues of the user.

5. The fabric (100) according to claim 3, wherein the gripping members are made by an array of protrusions (6), the array of protrusions (6) comprising a plurality of protrusion lines (6) extending along a first direction (X), the protrusions (6) of two adjacent lines being disposed in a staggered manner.

6. The fabric (100) according to claim 6, wherein each protrusion (6) has a dimension (d) along the first direction (X) of about 4 mm and a dimension (d') along a second direction substantially perpendicular to the first direction (X) of about 2.5 mm.

7. The fabric (100) according to any of claims 1 to 7, wherein the smallest dimension of the depression regions (2) is comprised between 1 and 2.5 cm.

8. The fabric (100) according to any of claims 1 to 8, wherein the smallest dimension of the pressure regions (1) is comprised between 0.5 and 2.5 cm.

9. The fabric (100) according to any of claims 1 to 9, wherein each of the pressure regions and each of the depression regions have the shape of a strip.

10. The fabric (100) according to any of claims 1 to 10, wherein at least one plurality of alternations of depression regions (2) and pressure regions (1) is knitted on a large cylinder circular knitting machine in a cut and sewn mode.

11. A compression garment (200) adapted to perform on a user a massage of the type palpate-roll, **characterized in that** the garment (200) comprises at least one portion formed of a fabric (100) according to any of claims 1 to 11.

12. The garment (200) according to claim 12, wherein the plurality of alternations of the pressure regions (1) and depression regions (2) are configured to compress the biological tissues of the user with a pressure comprised between 3 and 15 mm Hg and preferably between 7 and 9 mm Hg.

13. The garment (200) according to any of claims 12 to 13, wherein the garment (200) is a thigh strap, an arm cuff, a thigh cuff, or tights.

14. The garment (200) according to any of claims 12 to 14, wherein the pressure regions (1) have a generally annular shape.
